# EUROPEAN PATENT SPECIFICATION

(11) **EP 3 787 657 B1**
(45) Date of publication and mention of the grant of the patent: **30.08.2023**
(21) Application number: 19796025.5
(22) Date of filing: 30.04.2019
(51) Int. Cl.: A61K 36/14, A61K 36/185, A61K 36/21, A61P 13/12

(54) **HERBAL MEDICINES FOR TREATING POLYCYSTIC KIDNEY DISEASES**
PFLANZLICHE ARZNEIMITTEL ZUR BEHANDLUNG POLYZYSTISCHER NIERENERKRANKUNGEN
MÉDICAMENTS À BASE D'HERBES POUR LE TRAITEMENT DE MALADIES POLYKYSTIQUES DES REINS

(30) Priority: 30.04.2018 US 201862664709 P
(43) Date of publication of application: 10.03.2021
(73) Proprietor: Yarnell, Eric, Everett WA 98208 (US)
(72) Inventor: Yarnell, Eric, Everett WA 98208 (US)
(74) Representative: Hoffmann Eitle
(86) International application number: PCT/US2019/030005
(87) International publication number: WO 2019/213138

(56) References cited:
- US-A- 4 499 086
- Claudia Keel: "Red Root Ceanothus Americana, Ceanothus SPP", ArborVitae School of Traditional Herbalism - Article, pages 1-7, XP009523677,
- Anonymous: "Red Root (Ceanothus americana)", newwayherbs , 2 July 2017 (2017-07-02), pages 1-2, XP055743073, Retrieved from the Internet: URL:https://web.archive.org/web/2017070221 0958/http://store.newwayherbs.com/red-root -ceanothus-americana-p264.aspx [retrieved on 2019-06-21]
- Anonymous: "Tincture", Wikipedia , 4 July 2017 (2017-07-04), pages 1-3, XP055652157, Retrieved from the Internet: URL:https://en.wikipedia.Org/w/index.php?t itle=Tincture&oldid=789033862 [retrieved on 2019-06-21]

## Description

### BACKGROUND

### Technical Field

The present disclosure generally relates to compounds and methods for treating poly cystic kidney diseases.

### Description of the Related Art

Polycystic kidney diseases (PKDs) are rare conditions for which existing treatments are either poorly effective or very invasive and expensive. There is still no known treatment for the actual cause of the disease. Autosomal dominant (AD) and autosomal recessive (AR) PKD are the two main forms of this condition. Both are considered orphan diseases in the United States, affecting fewer than 200,000 people, though many estimates put the total number of patients at more than this threshold. It is the fourth leading cause of end-stage renal disease in North America. Approximately 2,500 people with PKD start on dialysis or have a kidney transplant every year in the United States [Descriptive epidemiology of ADPKD in the United States: Final study report. National ambulatory medical care survey (NAMCS), Centers for Disease Control National Center for Health Statistics. 2012-4].

PKDs are characterized by relentless growth of benign cysts in renal tissue, which mechanically crush surrounding tissue. Over time, this leads to progressive renal failure. Ultimately, either dialysis or renal transplant is required to keep patients alive. This is extremely expensive and subjects people with PKD to significant suffering and risks as well as many adverse effects. There are not currently enough kidneys available to supply everyone who needs one, so many patients with PKDs end up on chronic hemodialysis.

ARPKD is caused by mutations in the gene *PKHD1,* found at cytogenetic location 6p12.2 which codes for a protein known as polyductin or fibrocystin. ARPKD is estimated to occur in 1 in 20,000 live births in the USA. Very often, oligohydramnios, bilateral renal enlargement, and absence of urine the fetal bladder are seen *in utero* in the worst effected patients. Otherwise many patients with ARPKD present with liver problems due to congenital hepatic fibrosis, and kidney problems are only detected secondarily. ARPKD progresses much more rapidly than ADPKD, with most patients presenting in childhood and dying before reaching adulthood.

ADPKD has two types. Type 1 is due to mutations in the *ADPKD1* gene at cytogenetic location 16p13.3 and codes for a protein known as polycystin-1. Type 2 is due to mutations in the *ADPKD2* gene at cytogenetic location 4q22.1 that codes for a protein known as polycystin-2. Approximately 75-85% of ADPKD patients have type 1 disease. Approximately 10% of ADPKD cases arise because of a spontaneous mutation; the rest inherit the mutation from a parent. Type 1 ADPKD is generally more severe than type 2. Autopsy studies suggest the incidence of ADPKD is 1 in 400 to 1 in 1,000 people in the USA, with up to 12 million people affected worldwide, making it the most common potentially lethal monogenic disorder. The exact function of the polycystins has still not been determined, and the cause of mutations within the polycystins which results in ADPKD remains to be elucidated.

Most ADPKD patients are asymptomatic for long periods, until ultimately the cysts become large enough to cause changes in routine laboratory tests (most notably serum creatinine or urine protein), which can lead to performance of an ultrasound or other imaging that readily diagnoses the problem.

Currently the only drugs licensed by the U.S. Food and Drug Administration (U.S. FDA) for treatment of PKDs are tolvaptan and lixivaptan, which are non-peptide, small molecule agents that act as competitive vasopressin receptor type 2 antagonists. Tolvaptan and lixivaptan slow cyst growth by 50% compared to placebo while decreasing progression of kidney failure by approximately 30% in large clinical trials [Torres VE, et al. (2012) "Tolvaptan in patients with autosomal dominant polycystic kidney disease" N Engl J Med 367(25):2407]. The U.S. FDA has restricted use of tolvaptan to no more than 30 days continuously due to concerns about severe liver injury. Given the ongoing nature of PKD, this makes it highly unlikely these drugs would be safe. Additionally, at best, they only slow progression of PKD.

Many other treatments have been suggested as potential therapies, but all have failed so far. Perhaps the most spectacular were the mammalian target of rapamycin (mTOR) inhibitor drugs sirolimus and everolimus. Many lines of evidence suggest the mTOR promotes cyst growth in people with PKDs, and studies in animal models were promising. However, human clinical trials of both agents found no clinical benefit and even some evidence of harm [Serra AL, et al. (2010) "Sirolimus and kidney growth in autosomal dominant polycystic kidney disease" N Engl J Med 363(9):820-9; Walz G, et al. (2010) "Everolimus in patients with autosomal dominant polycystic kidney disease" N Engl J Med 363(9):830-40].

Drinking large amounts of water has also been posited as a way to delay cyst progression, and it seemed to have this effect in rodent models of PKD [Nagao S, et al. (2006) "Increased water intake decreases progression of polycystic kidney disease in the PCK rat" J Am Soc Nephrol 17(8):2220-7]. Unfortunately, the one human trial of this approach found the opposite, with signs of increased urine protein loss in patients assigned to drink large amounts of water compare to those drinking usual amounts [Higashihara E, et al. (2014) "Does increased water intake prevent disease progression in autosomal dominant polycystic kidney disease?" Nephrol Dial Transplant 29(9):1710-9].

Pravastatin is a well-known drug used primarily for lowering cholesterol levels to reduce cardiovascular disease risks. A single clinical trial in children with ADPKD showed that it was able to modestly slow progression of cyst growth [Cadnapaphornchai MA, et al. (2014) "Effect of pravastatin on total kidney volume, left ventricular mass index, and microalbuminuria in pediatric autosomal dominant polycystic kidney disease" Clin J Am Soc Nephrol 9(5):889-96]. This is not an U.S. FDA-approved use of this or any other statin drug, and research in adult patients is clearly needed.

The drug metformin, known as an insulin sensitizing agent, activates 5'-adenosine monophosphate-activated protein kinase (AMPK). Its use has been associated with reduction in cyst formation in animal models of ADPKD [Chang MY, et al. (2017) "Metformin inhibits cyst formation in a zebrafish model of polycystin-2 deficiency" Sci Rep 7(1):7161.]. The natural product berberine, which is believed to act by a similar mechanism as metformin, has shown similar efficacy in preclinical research [Bonon A, et al. (2013) "Berberine slows cell growth in autosomal dominant polycystic kidney disease cells" Biochem Biophys Res Commun 441(3):668-74]. No human clinical trials have been reported yet on the efficacy of either of these products for PKD.

Garren et al. (US 4,499,086) relates to compositions for the alleviation and curing of poison oak irritation and inflammation.

In summary, there is no known treatment that actually reverses any form of PKD. A handful of agents may mildly slow progression, but there is still an urgent need for effective treatment to shrink or eliminate cysts in these all too common serious diseases. The present disclosure fulfills these needs and provides further related advantages.

### BRIEF SUMMARY

The present invention relates to a composition comprising an extract of *Phytolacca americana, Fouquieria splendens, Parietaria judaica,* and a species of the *Ceanothus* genus and a composition comprising an extract of *Phytolacca americana, Fouquieria splendens, Parietaria judaica,* and a species of the *Ceanothus* genus for use in a method for treating polycystic kidney disease or polycystic ovarian syndrome. Further aspects of the invention are defined in the claims.

### DETAILED DESCRIPTION

In the following description, certain specific details are set forth in order to provide a thorough understanding of various aspects of the disclosure. However, one skilled in the art will understand that the disclosure may be practiced without these details.

Unless the context requires otherwise, throughout the present specification and claims, the word "comprise" and variations thereof, such as, "comprises" and "comprising" are to be construed in an open, inclusive sense, that is, as "including, but not limited to".

### Compositions

As detailed above, one particular aspect provides a composition comprising extracts of *Phytolacca americana, Fouquieria splendens, Parietaria judaica,* a species of the *Ceanothus* genus. Certain aspects comprise a pharmaceutically acceptable carrier or excipient and extracts of *Phytolacca americana, Fouquieria splendens, Parietaria judaica,* and a species of the *Ceanothus* genus.

In certain aspects, the composition comprises 4 extracts. In some aspects, the composition comprises 4 or more extracts. In certain aspects, the composition comprises an extract of *Phytolacca americana, Fouquieria splendens, Parietaria judaica,* and a species of the *Ceanothus* genus. Examples of a species of the *Ceanothus* genus include *Ceanothus greggii, Ceanothus cuneatus, Ceanothus fendleri,* and *Ceanothus integerrimus.* In some specific aspects, the species of the *Ceanothus* genus is *Ceanothus greggii.*

In some aspects, the pharmaceutical composition is formulated for oral administration. In other aspects, the pharmaceutical composition is formulated for injection. Suitable routes of administration include, but are not limited to, oral, intravenous, rectal, aerosol, parenteral, ophthalmic, pulmonary, transmucosal, transdermal, vaginal, otic, nasal, and topical administration. In addition, by way of example only, parenteral delivery includes intramuscular, subcutaneous, intravenous, intramedullary injections, as well as intrathecal, direct intraventricular, intraperitoneal, intralymphatic, and intranasal injections.

The composition can be prepared according to methods known in the art and is not particularly limited with respect to the methods used for extraction. Accordingly, in certain aspects, the extract is a water-ethanol extract. In some aspects, the extract is a vegetable glycerin extract. In some aspects, the extract is an oil extract. In some aspects, the extract is an extract of an organic solvent, including but not limited to alcohols, esters, and ethers. The organic solvent may also include acetonitrile, dichloromethane, ethanol, methanol, diethyl ether, ethyl acetate, acetone, DMSO and the like.

Additionally, plant parts from which the extracts were taken can be included in the composition. In certain aspects, powders or crude dried plant parts of *Phytolacca americana, Fouquieria splendens, Parietaria judaica,* a species of the *Ceanothus* genus or combinations thereof. In some aspects, the composition further comprises crushed fresh plant parts *Phytolacca americana, Fouquieria splendens, Parietaria judaica,* a species of the *Ceanothus* genus (*e.g*., *Ceanothus greggii, Ceanothus cuneatus, Ceanothus fendleri,* and *Ceanothus integerrimus)* or combinations thereof.

In some aspects, the concentration of the *Phytolacca americana* extract ranges from about 10-30% (*e.g*., about 10%, about 15%, about 20%, about 25% or about 30%) by volume of the composition.

In some more specific aspects, the concentration of the extract of the species of the *Ceanothus* genus (*e.g*., *Ceanothus greggii*) ranges from about 20-40% (*e.g*., about 20% , about 25%, about 30%, about 35% or about 40%) by volume of the composition. In some aspects, the concentration of the *Fouquieria splendens* extract ranges from about 1-20% (*e*.*g*., about 5%, about 10%, about 12%, about 15% or about 20%) by volume of the composition.

In some aspects, the concentration of the *Parietaria judaica* extract ranges from about 40-60% (*e.g*., about 40%, about 45%, about 50%, about 55% or about 60%) by volume of the composition.

In certain more specific aspect, the concentration of the *Phytolacca Americana* extract ranges from about 10-20%, the concentration of the extract of the species of the *Ceanothus* genus (*e.g*., *Ceanothus greggii*) ranges from about 20-40%, the concentration of the *Fouquieria splendens* extract ranges from about 1-20%, the concentration of the *Parietaria judaica* extract ranges from about 40-60% by volume of the composition.

In other more specific aspects, the concentration of the *Phytolacca Americana* extract is about 20%, the concentration of the extract of the species of the *Ceanothus* genus (*e.g*., *Ceanothus greggii*) is about 30%, the concentration of the *Fouquieria splendens* extract is about 10%, the concentration of the *Parietaria judaica* extract ranges from about 50% by volume of the composition.

In one aspect, the extract(s) are formulated in an aqueous solution. In specific aspects, the aqueous solution is selected from, by way of example only, a physiologically compatible buffer, such as Hank's solution, Ringer's solution, or physiological saline buffer. In specific aspects, such solutions include physiologically compatible buffers and/or excipients.

In another aspect, extracts described herein are formulated for oral administration. Compounds described herein are formulated by combining the active extracts with, e.g., pharmaceutically acceptable carriers or excipients. In various aspects, the extracts described herein are formulated in oral dosage forms that include, by way of example only, tablets, powders, pills, dragees, capsules, liquids, gels, syrups, elixirs, slurries, suspensions and the like.

In certain aspects, pharmaceutical preparations for oral use are obtained by mixing one or more solid excipient with one or more of the extracts (or dried powder form thereof) described herein, optionally grinding the resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as: for example, maize starch, wheat starch, rice starch, potato starch, gelatin, gum tragacanth, methylcellulose, microcrystalline cellulose, hydroxypropylmethylcellulose, sodium carboxymethylcellulose; or others such as: polyvinylpyrrolidone (PVP or povidone) or calcium phosphate. In specific aspects, disintegrating agents are optionally added. Disintegrating agents include, by way of example only, cross-linked croscarmellose sodium, polyvinylpyrrolidone, agar, or alginic acid or a salt thereof such as sodium alginate.

In one aspect, dosage forms, such as dragee cores and tablets, are provided with one or more suitable coating. In specific aspects, concentrated sugar solutions are used for coating the dosage form. The sugar solutions, optionally contain additional components, such as by way of example only, gum arabic, talc, polyvinylpyrrolidone, carbopol gel, polyethylene glycol, and/or titanium dioxide, lacquer solutions, and suitable organic solvents or solvent mixtures. Dyestuffs and/or pigments are also optionally added to the coatings for identification purposes. Additionally, the dyestuffs and/or pigments are optionally utilized to characterize different combinations of active compound doses.

### Administration

A composition described herein above can be administered according to any of the methods of administration described herein. In particular, certain aspects provide a composition comprising extracts from the group consisting of *Phytolacca americana, Fouquieria splendens, Parietaria judaica,* and a species of the *Ceanothus* genus (*e.g.*, *Ceanothus greggii, Ceanothus cuneatus, Ceanothus fendleri,* and *Ceanothus integerrimus)* for use in a method for treating polycystic kidney disease. The extracts may be formulated together or separately according to methods known in the art.

In certain aspects, the administration is oral. In some related aspects, the extract is a water-ethanol extract. In some aspects, the composition further comprises powders or crude dried plant parts of *Phytolacca americana, Fouquieria splendens, Parietaria judaica,* a species of the *Ceanothus* genus (*e.g., Ceanothus greggii, Ceanothus cuneatus, Ceanothus fendleri,* and *Ceanothus integerrimus)* or combinations thereof.

The compositions are effective in a variety of cystic diseases. Accordingly, in certain specific aspects, the polycystic kidney disease is autosomal dominant polycystic kidney disease, for example, type 1 or type 2. In other aspects, the polycystic kidney disease is autosomal recessive polycystic kidney disease.

Other aspects provide a composition comprising extracts from the group consisting of *Phytolacca americana, Fouquieria splendens, Parietaria judaica* and a species of the *Ceanothus* genus (*e.g*., *Ceanothus greggii, Ceanothus cuneatus, Ceanothus fendleri,* and *Ceanothus integerrimus*) for use in a method for treating a polycystic ovarian syndrome.

In more specific aspects of the foregoing, the administering is oral administration. In some aspects of the foregoing, the extract is a water-ethanol extract.

In certain related aspects, the composition further comprises powders or crude dried plant parts of *Phytolacca americana, Fouquieria splendens, Parietaria judaica* and a species of the *Ceanothus* genus (*e.g*., *Ceanothus greggii, Ceanothus cuneatus, Ceanothus fendleri,* and *Ceanothus integerrimus*) or combinations thereof.

Administration of the composition may continue as long as necessary. In some aspects, a composition is administered for more than 1, 2, 3, 4, 5, 6, 7, 14, or 28 days. In some aspects, a composition is administered for less than 28, 14, 7, 6, 5, 4, 3, 2, or 1 day. In some aspects, a composition is administered chronically on an ongoing basis, e.g., for the treatment of chronic effects.

In some aspects, the composition is administered in dosages. Due to intersubject variability in compound pharmacokinetics, individualization of dosing regimen is provided in certain aspects. Dosing for a composition of aspects of this disclosure may be found by routine experimentation in light of the instant disclosure and/or can be derived by one of ordinary skill in the art.

In some aspects, the composition is formulated into pharmaceutical compositions. In specific aspects, pharmaceutical compositions are formulated in a conventional manner using one or more physiologically acceptable carriers comprising excipients and auxiliaries which facilitate processing of the active compounds into preparations which can be used pharmaceutically. Proper formulation is dependent upon the route of administration chosen. Any pharmaceutically acceptable techniques, carriers, and excipients are used as suitable to formulate the pharmaceutical compositions described herein: Remington: The Science and Practice of Pharmacy, Nineteenth Ed (Easton, Pa.: Mack Publishing Company, 1995); Hoover, John E., Remington's Pharmaceutical Sciences, Mack Publishing Co., Easton, Pennsylvania 1975; Liberman, H.A. and Lachman, L., Eds., Pharmaceutical Dosage Forms, Marcel Decker, New York, N.Y., 1980; and Pharmaceutical Dosage Forms and Drug Delivery Systems, Seventh Ed. (Lippincott Williams & Wilkins1999).

### EXAMPLE 1

### PREPARATION OF COMPOSITIONS

A representative composition was prepared by extracting a fresh plant mixture by coarsely chopping plant parts and macerating the resultant mixture in a solvent mixture of water, ethanol, and vegetable glycerin. Specifically, 600 g of fresh, coarsely chopped *Phytolacca americana* (poke) root was mixed with 900 mL ethanol, 180 mL water, and 120 mL vegetable glycerin and macerated for 30 days. Of *Ceanothus greggii* (red root), 470 g of coarsely chopped fresh root was mixed with 850 mL ethanol, 420 mL water, and 140 mL vegetable glycerin and macerated for 30 days. Of *Fouquieria splendens* (ocotillo), 470 g of coarsely chopped fresh bark was mixed with 1,270 mL ethanol and 140 mL vegetable glycerin and macerated for 30 days. Of *Parietaria judaica* (pellitory-of-the-wall), 470 g of coarsely chopped leaf, stem, and flower were mixed with 560 mL ethanol, 700 mL water, and 140 mL vegetable glycerin.

The resultant composition in each case was then pressed to excrete the liquid extract and strained to remove any residual solid material. All four extracts were then combined in a ratio of 20% *Phytolacca americana,* 30% *Ceanothus greggii,* 10% *Fouquieria splendens,* and 50% *Parietaria Judaica.*

### EXAMPLE 2

### ADMINISTRATION OF REPRESENTATIVE COMPOSITIONS (1^{ST} STUDY)

Shrinkage of kidney and other cysts caused by ADPKDs was observed upon administration of the composition in two patients (numbers A1 and A4) who had pre- and post-total kidney volume (TKV) assessed. The shrinkage represents halting and reversal of progression of ADPKD. All four patients report an improvement in their estimated glomerular filtration rate (eGFR) since starting treatment with a minimum six-month follow-up. eGFR is the standard for assessing overall kidney function and its improvement is a clear indication that as their kidneys and cysts are shrinking and damage is being repaired.

**Table A1. Representative results from administration of compositions of the present disclosure**

| **Patient number** | **Age** | | **SexRace** | **CKD stage** | **eGFR pre-tx (mL/min)** | **eGFR post-tx (mL/min)** | **TKV pre-tx (mL)** | **TKV post-tx (mL)** |
|---|---|---|---|---|---|---|---|---|
| A1 | 35 | M | P | G3a | - | - | 1038 | 840 |
| A3 | 35 | F | W | G2b | 72.4 | - | ^{∗} | - |
| A4 | 37 | M | W | G2b | 72 | - | 783.5 | - |
| A7 | 29 | M | P | G3a | - | - | - | - |

| | | | | | | | | |
|---|---|---|---|---|---|---|---|---|
| Abbreviations: Sex: F = female, M = male Race: P = Persian, W = White CKD = chronic kidney disease eGFR = estimated glomerular filtration rate TKV = total kidney volume. * = both kidneys were 15.3 cm in the longitudinal axis | | | | | | | | |

### EXAMPLE 3

### ADMINISTRATION OF REPRESENTATIVE COMPOSITIONS (2^{ND} STUDY)

Shrinkage or stabilization of kidney and other cysts caused by ADPKD was observed upon administration of the composition in two patients (numbers B1 and B5) who had pre- and post-total kidney volume (TKV) assessed. This represented a halting and reversal of progression of ADPKD. The one other patient with pre- and post-TKV measurements had an approximate 11% increase, but treatment duration was only three months. TKV is recognized in the art as a biomarker for efficacy of therapies for PKD. Eight of 11 treated patients had an improvement or stabilization in their estimated glomerular filtration rate (eGFR) since starting treatment with a minimum three-month follow-up (allowing for an error of ±1 mL/min in eGFR measurement). eGFR is the standard for assessing overall kidney function and its improvement is an indication that kidneys and cysts are shrinking and damage is being repaired. Even patients who had worsening of their eGFR over time (particularly patients B2 and B6), their initial kidney disease (stage G4) was relatively severe (and patient B6's kidneys were enormous on initial imaging, though no post-treatment sizing information was available) and the degree of progression was remarkably low for such situations. The deterioration of patient B1's eGFR was most mysterious, as imaging showed his TKV significantly improved with treatment.

**Table B1. Representative results from administration of compositions of the present disclosure**

| **Patient number** | **Age** | **Sex, Race** | **CKD stage (baseline)** | **eGFR pre-tx (mL/min)** | **eGFR post-tx (mL/min)** | **TKV pre-tx (mL)** | **TKV post-tx(mL)** | **Tx duration (years)** |
|---|---|---|---|---|---|---|---|---|
| B1 | 35 | M, P | G2 A1 | 84 | 76 | 1038 | 840 | 2.75 |
| B2 | 47 | F, W | G3b A1 | 37 | 29 | - | - | 3.5 |
| B3 | 37 | M, W | G2 | 72 | 74 | 783.5 | - | 0.5 |
| B4 | 38 | M, AA | G3a | 58 | 67 | 1153 | - | 0.5 |
| B5 | 42 | M, W | G3b A3 | 42 | 46 | 3140 | 3151 | 1 |
| B6 | 63 | M, W | G4 | 23 | 20 | 5240 | - | 2.5 |
| B7 | 65 | M, W | G3b A1a | 40 | 51 | - | - | 0.67 |
| B8 | 66 | F, W | G2 | 72 | 74 | 524 | 580 | 0.25 |
| B9 | 46 | M, W | G3b | 31 | 30 | - | - | 1.5 |
| B10 | 71 | M, AA | G5d | 4 | 5 | - | - | 1 |
| B11 | 38 | F, W | G2A2 | 66 | 66 | 1773 | - | 1.25 |

| | | | | | | | | |
|---|---|---|---|---|---|---|---|---|
| Abbreviations: Sex: F = female, M = male Race: AA = African American, P = Persian, W = White Stage: A = albumin-based, d = on dialysis, G = glomerular-filtration rate-based CKD = chronic kidney disease eGFR = estimated glomerular filtration rate TKV = total kidney volume | | | | | | | | |

## Claims

1. A composition comprising an extract of *Phytolacca americana, Fouquieria splendens, Parietaria judaica,* and a species of the *Ceanothus* genus.

2. The composition of claim 1, wherein the extract is a water-ethanol extract.

3. The composition of any one of claims 1-2, wherein the composition further comprises powders or crude dried plant parts of *Phytolacca americana, Fouquieria splendens, Parietaria judaica,* a species of the *Ceanothus* genus or combinations thereof.

4. The composition of any one of claims 1-3, wherein the species of the *Ceanothus* genus is selected from the group consisting of *Ceanothus greggii, Ceanothus cuneatus, Ceanothus fendleri,* and *Ceanothus integerrimus.*

5. The composition of any one of claims 1-4, wherein the concentration of the *Phytolacca americana* extract ranges from about 10-30% by volume of the composition.

6. The composition of any one of claims 1-5, wherein:
a) the concentration of the extract of the species of the *Ceanothus* genus ranges from about 20-40% by volume of the composition;
b) the concentration of the *Fouquieria splendens* extract ranges from about 1-20% by volume of the composition; or
c) the concentration of the *Parietariajudaica* extract ranges from about 40-60% by volume of the composition.

7. The composition of any one of claims 1-6, wherein the concentration of the *Phytolacca americana* extract ranges from about 10-20%, the concentration of the extract of the species of the *Ceanothus* genus ranges from about 20-40%, the concentration of the *Fouquieria splendens* extract ranges from about 1-20%, and the concentration of the *Parietaria judaica* extract ranges from about 40-60% by volume of the composition.

8. The composition of any one of claims 1-7, wherein the concentration of the *Phytolacca Americana* extract is about 20%, the concentration of the extract of the species of the *Ceanothus* genus is about 30%, the concentration of the *Fouquieria splendens* extract is about 10%, and the *Parietaria judaica* extract ranges from about 50% by volume of the composition.

9. The composition of any one of claims 1-8, wherein the species of the *Ceanothus* genus is *Ceanothus greggii.*

10. A composition comprising an extract of *Phytolacca americana, Fouquieria splendens, Parietaria judaica,* and a species of the *Ceanothus* genus for use in a method for treating polycystic kidney disease or polycystic ovarian syndrome.

11. The composition for use of claim 10, wherein administering is oral administration.

12. The composition for use of any one of claims 10-11, wherein the extract is a water-ethanol extract.

13. The composition for use of any one of claims 10-12, wherein the composition further comprises powders or crude dried plant parts of *Phytolacca americana, Fouquieria splendens, Parietaria judaica,* a species of the *Ceanothus* genus or combinations thereof.

14. The composition for use of any one of claims 10-13, wherein the polycystic kidney disease is autosomal dominant polycystic kidney disease.

15. The composition for use of any one of claims 10-14, wherein the polycystic kidney disease is autosomal recessive polycystic kidney disease.

## Patentansprüche

1. Zusammensetzung, umfassend ein Extrakt aus *Phytolacca americana, Fouquieria splendens, Parietaria judaica* und einer Spezies der Ceanothus-Gattung.

2. Zusammensetzung gemäß Anspruch 1, wobei das Extrakt ein Wasser-Ethanol-Extrakt ist.

3. Zusammensetzung gemäß irgendeinem der Ansprüche 1-2, wobei die Zusammensetzung ferner Pulver oder rohe getrocknete Pflanzenteile von *Phytolacca americana, Fouquieria splendens, Parietaria judaica,* einer Spezies der Ceanothus-Gattung oder Kombinationen davon umfasst.

4. Zusammensetzung gemäß irgendeinem der Ansprüche 1-3, wobei die Spezies der Ceanothus-Gattung ausgewählt ist aus der Gruppe bestehend aus *Ceanothus greggii, Ceanothus cuneatus, Ceanothus fendleri,* und *Ceanothus integerrimus.*

5. Zusammensetzung gemäß irgendeinem der Ansprüche 1-4, wobei die Konzentration des *Phytolacca americana-*Extrakts im Bereich von etwa 10-30% des Volumens der Zusammensetzung liegt.

6. Zusammensetzung gemäß irgendeinem der Ansprüche 1-5, wobei:
a) die Konzentration des Extrakts der Spezies der *Ceanothus*-Gattung im Bereich von etwa 20-40% des Volumens der Zusammensetzung liegt;
b) die Konzentration des Extrakts von *Fouquieria splendens* im Bereich von etwa 1-20% des Volumens der Zusammensetzung liegt; oder
c) die Konzentration des *Parietaria judaica*-Extrakts im Bereich von etwa 40-60% des Volumens der Zusammensetzung liegt.

7. Zusammensetzung gemäß irgendeinem der Ansprüche 1-6, wobei die Konzentration des *Phytolacca americana-*Extrakts im Bereich von etwa 10-20%, die Konzentration des Extrakts der Spezies der Ceanothus-Gattung im Bereich von etwa 20-40%, die Konzentration des *Fouquieria splendens* Extraktes bei etwa 1-20% und die Konzentration des *Parietaria judaica*-Extraktes bei von etwa 40-60%, bezogen auf das Volumen der Zusammensetzung liegt.

8. Zusammensetzung gemäß irgendeinem der Ansprüche 1-7, wobei die Konzentration des *Phytolacca Americana-*Extrakts etwa 20%, die Konzentration des Extrakts der Spezies der Ceanothus-Gattung etwa 30%, die Konzentration des Extrakts von *Fouquieria splendens* etwa 10% und das Extrakt von *Parietaria judaica* etwa 50% des Volumens der Zusammensetzung beträgt.

9. Zusammensetzung gemäß irgendeinem der Ansprüche 1-8, wobei die Spezies der *Ceanothus*-Gattung *Ceanothus greggii* ist.

10. Zusammensetzung, umfassend ein Extrakt aus *Phytolacca americana, Fouquieria splendens, Parietaria judaica* und einer Spezies der Ceanothus-Gattung zur Verwendung in einem Verfahren zur Behandlung von polyzystischer Nierenerkrankung oder polyzystischem Ovarialsyndrom.

11. Zusammensetzung zur Verwendung gemäß Anspruch 10, wobei die Verabreichung eine orale Verabreichung ist.

12. Zusammensetzung zur Verwendung gemäß irgendeinem der Ansprüche 10-11, wobei das Extrakt ein Wasser-Ethanol-Extrakt ist.

13. Zusammensetzung zur Verwendung gemäß irgendeinem der Ansprüche 10-12, wobei die Zusammensetzung ferner Pulver oder rohe getrocknete Pflanzenteile von *Phytolacca americana, Fouquieria splendens, Parietaria judaica,* einer Spezies der Ceanothus-Gattung oder Kombinationen davon umfasst.

14. Zusammensetzung zur Verwendung gemäß irgendeinem der Ansprüche 10-13, wobei die polyzystische Nierenerkrankung eine autosomal dominante polyzystische Nierenerkrankung ist.

15. Zusammensetzung zur Verwendung gemäß irgendeinem der Ansprüche 10-14, wobei die polyzystische Nierenerkrankung eine autosomal rezessive polyzystische Nierenerkrankung ist.

## Revendications

1. Composition comprenant un extrait de *Phytolacca americana, Fouquieria splendens, Parietaria judaica* et une espèce du genre *Ceanothus.*

2. Composition selon la revendication 1, dans laquelle l'extrait est un extrait eau-éthanol.

3. Composition selon l'une quelconque des revendications 1 à 2, dans laquelle la composition comprend en outre des poudres ou des parties de plantes séchées brutes de *Phytolacca americana, Fouquieria splendens, Parietaria judaica,* une espèce du genre *Ceanothus* ou des combinaisons de ceux-ci.

4. Composition selon l'une quelconque des revendications 1 à 3, dans laquelle l'espèce du genre *Ceanothus* est choisie dans le groupe composé de *Ceanothus greggii, Ceanothus cuneatus, Ceanothus fendleri* et *Ceanothus integerrimus.*

5. Composition selon l'une quelconque des revendications 1 à 4, dans laquelle la concentration d'extrait de *Phytolacca americana* est comprise dans la plage d'environ 10 à 30 % en volume de la composition

6. Composition selon l'une quelconque des revendications 1 à 5, dans laquelle :
a) la concentration d'extrait de l'espèce du genre *Ceanothus* est comprise dans la plage d'environ 20 à 40 % en volume de la composition ;
b) la concentration d'extrait de *Fouquieria splendens* est comprise dans la plage d'environ 1 à 20 % en volume de la composition ; ou
c) la concentration d'extrait de *Parietaria judaica* est comprise dans la plage d'environ 40 à 60 % en volume de la composition.

7. Composition selon l'une quelconque des revendications 1 à 6, dans laquelle la concentration d'extrait de *Phytolacca americana* est comprise dans la plage d'environ 10 à 20 %, la concentration d'extrait de l'espèce du genre *Ceanothus* est comprise dans la plage d'environ 20 à 40 %, la concentration d'extrait de *Fouquieria splendens* est comprise dans la plage d'environ 1 à 20 %, et la concentration d'extrait de *Parietaria judaica* est comprise dans la plage d'environ 40 à 60 % en volume de la composition.

8. Composition selon l'une quelconque des revendications 1 à 7, dans laquelle la concentration d'extrait de *Phytolacca Americana* est d'environ 20 %, la concentration d'extrait de l'espèce du genre *Ceanothus* est d'environ 30 %, la concentration d'extrait de *Fouquieria splendens* est d'environ 10 %, et la concentration d'extrait de *Parietaria judaica* est comprise dans la plage d'environ 50 % en volume de la composition.

9. Composition selon l'une quelconque des revendications 1 à 8, dans laquelle l'espèce du genre *Ceanothus* est *Ceanothus greggii.*

10. Composition comprenant un extrait de *Phytolacca americana, Fouquieria splendens, Parietaria judaica,* et une espèce du genre *Ceanothus* destinée à une utilisation dans un procédé de traitement de la maladie polykystique des reins ou le syndrome des ovaires polykystiques.

11. Composition pour une utilisation selon la revendication 10, dans laquelle l'administration est une administration orale.

12. Composition pour une utilisation selon l'une quelconque des revendications 10 à 11, dans laquelle l'extrait est un extrait eau-éthanol.

13. Composition pour une utilisation selon l'une quelconque des revendications 10 à 12, dans laquelle la composition comprend en outre des poudres ou des parties de plantes séchées brutes de *Phytolacca americana, Fouquieria splendens, Parietaria judaica,* une espèce du genre *Ceanothus* ou des combinaisons de ceux-ci.

14. Composition pour une utilisation selon l'une quelconque des revendications 10 à 13, dans laquelle la maladie polykystique des reins est une maladie polykystique des reins autosomique dominante.

15. Composition pour une utilisation selon l'une quelconque des revendications 10 à 14, dans laquelle la maladie polykystique des reins est une maladie polykystique des reins autosomique récessive.
